# EUROPEAN PATENT APPLICATION

(11) **EP 3 231 796 A1**
(43) Date of publication of application: **18.10.2017**
(21) Application number: 16382169.7
(22) Date of filing: 13.04.2016
(51) Int. Cl.: C07D 401/06, A61K 31/4709, A61P 33/10, A61P 33/00

(54) **A PROCESS FOR THE PREPARATION OF PYRVINIUM PAMOATE AND CRYSTALLINE FORMS THEREOF**

(71) Applicant: Urquima S.A., 08184 Palau Solità I Plegamans (ES)
(72) Inventor: DEL RÍO PERICACHO, José Luis, 08225 Terrassa (ES); MARTÍ VIA, Josep, 08023 Barcelona (ES); CÁNOVAS PAREDES, Antonio de Padua, 08551 Tona (ES)
(74) Representative: ZBM Patents - Zea, Barlocci & Markvardsen

(57) **Abstract**

The present invention relates to a process for the preparation of pyrvinium pamoate, which comprises reacting pyrvinium methyl sulphate salt of formula (II) with disodium pamoate of formula (III), in the presence of a base; to processes for the preparation of crystalline Form A and crystalline Form III of pyrvinium pamoate; as well as a process for the purification of crystalline Form III. It also relates to crystalline Forms A and III of pyrvinium pamoate and to pharmaceutical compositions containing them.

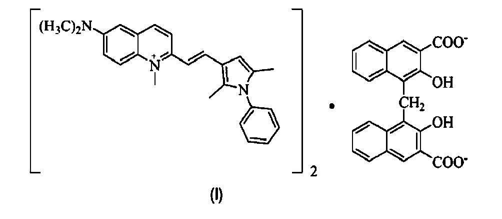

## Description

The present invention relates to a new process for the preparation of pyrvinium pamoate and a process for its purification. It also relates to new crystalline forms of pyrvinium pamoate and to processes for their preparation as well as to pharmaceutical composition containing them.

### STATE OF THE ART

Pyrvinium pamoate salt is the name of the compound (E)-6-(dimethylamino)-2-[2-(2,5-dimethyl-1-phenyl-1*H*-pyrrol-3-yl) ethenyl]-1-methyl-quinolinium pamoate salt of formula (I) whose chemical structure is the following:

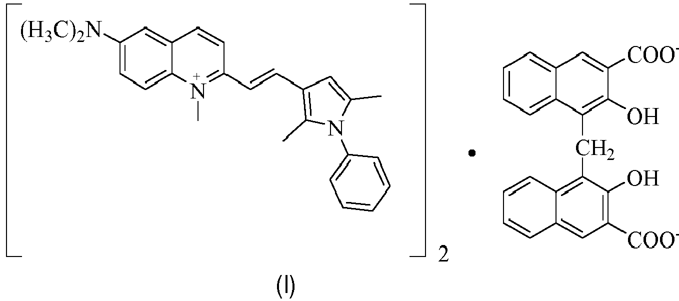

Pyrvinium pamoate salt of formula (I) (from now on called "pyrvinium pamoate") is an inhibitor of NADH-fumarate reductase (NADH-FR) activity in the anaerobic respiratory chain in mitochondria of parasitic worm, and therefore, it is effective against parasitic nematodes, including nematodes of the genera Enterobius and Stronglyloides. It is indicated as anthelmintic drug for the treatment of enterobiasis caused by Enterobius vermicularis.

The United States patent US2925417 discloses several processes for the preparation of pyrvinium pamoate. These processes are mainly based on the preparation of the pyrvinium pamoate from simple pyrvinium salts such as for example chloride and iodide salts. Furthermore, the PCT patent application WO2006078754 discloses the preparation of the pyrvinium phosphate salt and the pyrvinium sulphate salt from pyrvinium pamoate.

The different solid forms of a pharmaceutically active ingredient can have different characteristics, and offer certain advantages, for example with regard to stability, bioavailability, ease of formulation, ease of administration, among others. Since some solid forms are more adequate for one type of formulation, and other forms for other different formulations, the development of new solid forms allows for improving the characteristics of the pharmaceutical formulations comprising them. In addition, depending on the therapeutic indications, one or another pharmaceutical formulation may be preferred.

Especially desirable improvements/advantages of the new polymorphs form would include, for example, better stability, flowability, solubility, tractability, or compressibility, improvement of physicochemical properties in order to facilitate its manufacture or its formulation, to enhance the absorption and/or the bioavailability, being easily obtainable with more constant physicochemical properties, allowing more flexibility while formulating, or facilitating its formulation, better dispersibility properties, thus allowing better dispersion rates, especially if dispersed in an aqueous physiological surrounding, or reducing hygroscopicity, allowing new routes of administration.

Thus, there is a need to develop a more economical and more easily scaling process for the preparation of the pyrvinium pamoate and, particularly, for the preparation of solid forms of the pyrvinium pamoate.

### SUMMARY OF THE INVENTION

The inventors have found a new process for the preparation of the pyrvinium pamoate which comprises the use of the pyrvinium methyl sulphate salt of formula (II) as starting material and which presents several advantages, mainly in terms of yields, lower process costs, environmental impact, and at the same time allowing an easy industrialization.

An advantage of the process of the present invention is that it avoids the use of unsuitable reagents such as for example halogenated alkanes. Further, the process of the invention allows using a low amount of the pyrvinium methyl sulphate of formula (II) in relation to the amount of the disodium pamoate. This has a clear impact in the purity of the product obtained. Thus, another advantage of the process lies in the fact that the pyrvinium pamoate can be obtained with high yields and high purity, and therefore, the purification of the pyrvinium pamoate is simplified or even can be avoided, resulting in a simple and industrial scale-up process.

The process may further comprise the preparation of a new crystalline form of pyrvinium pamoate, named crystalline Form III that is obtained with high yields and high purity. The preparation of this crystalline form also has a clear impact in the purity of the product obtained. Finally, the process may also comprise additional purification steps which go through the formation of a new crystalline form of the pyrvinium pamoate, named crystalline Form A, which not only allow reducing the amount of the impurity named (E)-2-[2-(2,5-dimethyl-1-phenyl-1 H-pyrrol-3-yl)-vinyl]-1-methyl-6-methylamino-quinolinium pamoate but also of several other unknown impurities.

Therefore, an aspect of the invention relates to a process for the preparation of the pyrvinium pamoate, 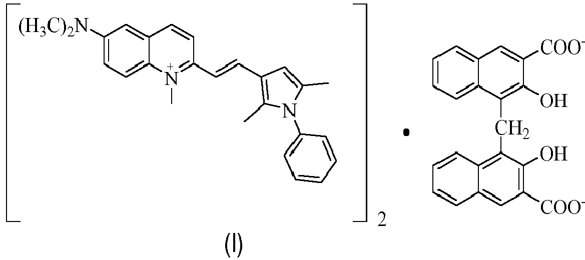 which comprises reacting pyrvinium methyl sulphate salt of formula (II), 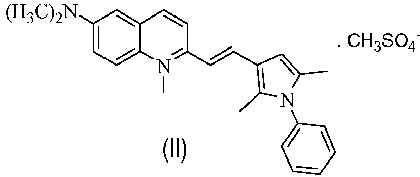 with disodium pamoate of formula (III), 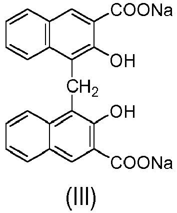 optionally, in the presence of a base.

A second aspect of the invention relates a process for the preparation of a crystalline Form A of pirvinium pamoate which comprises: (c) slurring the crystalline Form III of the pyrvinium pamoate with ethanol at reflux temperature for the necessary period of time to convert the crystalline Form III in crystalline Form A; and (d) cooling at a temperature comprised from 20°C to 30°C.

A third aspect of the invention relates to the crystalline Form A of pyrvinium pamoate characterized by having an X-ray diffractogram that comprises characteristic peaks at 2.8, 5.1, 7.2, 8.9, 9.6, 9.9, 15.9, 16.3, 17.9, 21.0 ± 0.2 degrees 2 theta measured in an X-ray diffractometer with Cu Kα radiation (1.5418 Å).

A fourth aspect of the invention relates to a process for the preparation of crystalline Form III of pyrvinium pamoate which comprises: (g) slurring the crystalline Form A of pyrvinium pamoate with a mixture of water and ethanol at reflux temperature for the necessary period of time to convert the crystalline Form A in crystalline Form III; (h) separating the crystalline Form III obtained in step (g) at a temperature comprised from 90°C to 95°C; and (i) washing the crystalline Form III of pyrvinium pamoate salt first with hot water at a temperature comprised from 90°C to 95°C and then with ethanol.

A fifth aspect of the invention relates to a process for the purification of crystalline Form III of pyrvinium pamoate which comprises preparing crystalline Form A of pyrvinium pamoate from crystalline Form III obtained by the process as defined in the second aspect of the invention and then transforming crystalline Form A into crystalline Form III by the process as defined in the fourth aspect of the invention.

A sixth aspect of the invention relates to the crystalline Form III of pyrvinium pamoate characterized by having an X-ray diffractogram that comprises characteristic peaks at 6.0, 8.6, 9.5, 10.3, 10.5, 14.3, 14.9, 16.6, 17.4, 18.1, 19.1, 19.7, 20.6, 21.5, 22.0, 22.6, 23.6, 24.9 ± 0.2 degrees 2 theta measured in an X-ray diffractometer with Cu Kα radiation (1.5418 Å).

Finally, a seventh aspect of the invention relates to a pharmaceutical composition comprising the crystalline form of pyrvinium pamoate as defined in the fifth and the sixth aspect of the invention, together with one or more pharmaceutically acceptable excipients or carriers.

### BRIEF DESCRIPTION OF THE DRAWINGS

FIG. 1 shows the powder X-ray diffraction pattern (intensity (counts) versus 2-theta angle (°)) of the crystalline Form A of pyrvinium pamoate.
FIG. 2 shows the infrared (IR) spectrum of the crystalline Form A of pyrvinium pamoate. The curve expresses the transmittance (T) versus the wavenumber value (cm⁻¹).
FIG. 3 shows the powder X-ray diffraction pattern (intensity (counts) versus 2-theta angle (°)) of the crystalline Form III of the pyrvinium pamoate.
FIG. 4 shows the infrared (IR) spectrum of the crystalline Form III of the pyrvinium pamoate. The curve expresses the transmittance (T) versus the wavenumber value (cm⁻¹).

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For the purposes of the invention, any ranges given include both the lower and the upper endpoints of the range. Ranges given, such as temperatures, times, and the like, should be considered approximate, unless specifically stated.

The term "molar ratio" refers to the relation of moles between the starting material and the reagent needed to perform the reaction. For example, the relation of moles between the moles of base and the moles of the pyrvinium methyl sulphate salt of formula (II); or the relation of moles between the pyrvinium methyl sulphate of formula (II) and the disodium pamoate of formula (III).

The term "volume ratio" refers to the relation of the volume of a first compound in relation to the volume of a second compound. For example, volume ratio between water and C₁-C₄ alcohol refers to the amount of water in relation to the amount of C₁-C₄ alcohol.

The term "alkyl" refers to a linear or branched hydrocarbon chain which contains the number of carbon atoms specified in the description or the claims.

The term "alcohol" refers to a hydrocarbon derivative in which one or more hydrogen atoms have been replaced by one or more -OH group. The term alcohol also includes glycol compounds. Examples of appropriate alcohols for the present invention include, but are not limited to, ethanol, 2-propanol (i.e., isopropanol), or n-propanol.

The term "reflux temperature" refers to the temperature at which the mixture boils under conditions in which the solvent vapour returns to the liquid mixture after condensation.

The term "therapeutic effective amount" refers to the amount of the active ingredient (pyrvinium pamoate) that provides the therapeutic effect after its application.

The term "pharmaceutically acceptable" refers to that excipients or carriers suitable for use in the pharmaceutical technology for preparing compositions with medical use.

For the purpose of the invention, the water content is determined by Karl Fisher titrimetry (KFT) as is expressed in % by weight of water in relation to the weight of the pyrvinium pamoate.

When values of characteristic peaks of an X-ray diffractogram are given it is said that are "approximate" values. It should be understood that the values are the ones shown in the corresponding lists or tables ± 0.2 degrees 2 theta measured in an X-ray diffractometer with Cu-Kα radiation λ=1.5406 Å.

As mentioned above, the first aspect of the present invention relates to a process for the preparation of pyrvinium pamoate, which comprises reacting pyrvinium methyl sulphate salt of formula (II), with disodium pamoate of formula (III).

In an embodiment, optionally in combination with one or more features of the various embodiments described above or below, the process is performed in the presence of a base. In another embodiment, optionally in combination with one or more features of the various embodiments described above or below, the base is selected from the group consisting of ammonia, (C₁-C₄ alkyl)₂-amines, (C₁-C₄ alkyl)₃-amines, cyclic amines, alkali metal hydroxides and alkaline earth metal hydroxides. Examples of amines suitable for the present invention can be diethyl amine, triethyl amine, piperidine, and sodium hydroxide. In a preferred embodiment, optionally in combination with one or more features of the various embodiments described above or below, the base is ammonia.

In an embodiment, optionally in combination with one or more features of the various embodiments described above or below, the process is that wherein the molar ratio between the base and the pyrvinium methyl sulphate salt of formula (II) is comprised from 0.32 to 6.51; preferably comprised from 0.32 to 1.95. In a particular embodiment, optionally in combination with one or more features of the various embodiments described above or below, the process is performed in the presence of ammonia wherein the molar ratio between ammonia and the pyrvinium methyl sulphate salt of formula (II) is comprised from 0.32 to 6.51; preferably the process is performed in the presence of ammonia wherein the molar ratio between ammonia and the pyrvinium methyl sulphate salt of formula (II) is comprised from 0.32 to 1.95.

In an embodiment, optionally in combination with one or more features of the various embodiments described above or below, the process is that wherein the molar ratio between the pyrvinium methyl sulphate of formula (II) and the disodium pamoate of formula (III) is comprised from 1.33 to 2.00; preferably comprised from 1.66 to 1.96. The term "molar ratio" has been used to express the stoichiometric amount in moles of each of the starting materials (compounds of formula (II) and (III)).

In an embodiment, optionally in combination with one or more features of the various embodiments described above or below, the process is performed in the presence of a solvent selected from water, C₁-C₄ alcohol, dimethylformamide, C₃-C₆ cyclic ethers and mixtures thereof. Examples of appropriate C₃-C₆ cyclic ethers include tetrahydrofurane and dioxane.

In an embodiment, optionally in combination with one or more features of the various embodiments described above or below, the process is performed in the presence of a mixture of water and C₁-C₄ alcohol, and then the process renders the crystalline Form III of the pyrvinium pamoate. Examples of appropriate C₁-C₄ alcohol as a solvent for the present invention include, but are not limited to, methanol, ethanol and isopropanol. In an embodiment, optionally in combination with one or more features of the various embodiments described above or below, the process is performed in the presence of a mixture of water and ethanol. In a particular embodiment, optionally in combination with one or more features of the various embodiments described above or below, the process is performed in a mixture of water and ethanol, wherein the volume ratio between the water and the C₁-C₄ alcohol is comprised from 40 to 3; preferably comprised from 25 to 10. Thus, a process for the preparation of crystalline Form III of the pyrvinium pamoate which comprises performing the process as defined in the first aspect of the invention in the presence of a mixture of water and C₁-C₄ alcohol is also part of the invention.

In an embodiment, optionally in combination with one or more features of the various embodiments described above or below, the process is performed at a reflux temperature of the solvent.

Typically, the pyrvinium pamoate obtained by the process of the first aspect of the invention has a chemical purity equal to or higher than 99.0 area %; preferably, equal to or higher than 99.3 area %. In an embodiment, wherein the process of the first aspect of the invention is performed in the presence of a mixture of water and C₁-C₄ alcohol, the process allows obtaining the crystalline Form III of pyrvinium pamoate having a chemical purity up to 99.5 area % measured by HPLC. The process of the invention further allows obtaining the crystalline Form III of pyrvinium pamoate with a water content equal to or less than 6.0% by weight. In a preferred embodiment, the water content is comprised from 4.0% to 6.0% by weight.

In an alternative embodiment, optionally in combination with one or more features of the various embodiments described above or below, wherein the process of the invention renders a crystalline Form III of pyrvinium pamoate having a water content higher than 6.0% by weight, then the process further comprises an additional step of drying the pyrvinium pamoate until having a water content equal to or less than 6.0% by weight. In an embodiment, optionally in combination with one or more features of the various embodiments described above or below, the additional drying step is performed at a temperature comprised from 80°C and 90°C; preferably comprised from 84°C and 88°C. In an embodiment, optionally in combination with one or more features of the various embodiments described above or below, the additional drying step is performed for the necessary period of time to dry the pyrvinium pamoate; preferably for a period of time comprised from 2 to 5 h; more preferably for 4 h. In a particular embodiment, optionally in combination with one or more features of the various embodiments described above or below, the additional drying step is performed at a temperature comprised from 80°C and 90°C for a period of time comprised from 2 to 5 h; preferably at a temperature comprised form 84°C and 88°C for 4 h. Drying may be performed by any method known in the art. Examples of drying methods appropriate for the present invention can be oven drying and vacuum desiccation.

In an embodiment, optionally in combination with one or more features of the various embodiments described above or below, the process of the invention further comprises first preparing the pyrvinium methyl sulphate of formula (II), then the process comprises: a) reacting a compound of formula (IV), 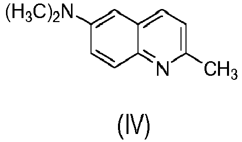 with dimethyl sulphate; and b) reacting the mixture obtained in step a) with a compound of formula (V) 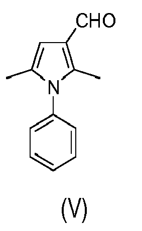 in the presence of a base.

In an embodiment, optionally in combination with one or more features of the various embodiments described above or below, step b) of the process of the invention is performed in the presence of a base selected from the group consisting of ammonia, (C₁-C₄ alkyl)₂-amines, (C₁-C₄ alkyl)₃-amines, cyclic amines and alkali metal hydrure. Examples of amines suitable for the present invention can be diethyl amine, diisopropyl amine, piperidine, pyrrolidine, hexamethyldisilazane, lithium hexamethyldisilazane, sodium hydride, butyl lithium, hexyl lithium, sodium methoxide and sodium ethoxide. In a preferred embodiment, optionally in combination with one or more features of the various embodiments described above or below, the base is piperidine. In an embodiment, optionally in combination with one or more features of the various embodiments described above or below, the process is that wherein the molar ratio between the base and the compound of formula (IV) is comprised from 0.39 to 1.94; preferably comprised from 0.58 to 0.97. In a particular embodiment, optionally in combination with one or more features of the various embodiments described above or below, the process is performed in the presence of piperidine wherein the molar ratio between piperidine and the compound of formula (IV) is comprised from 0.39 to 1.94; preferably the process is performed in the presence of piperidine wherein the molar ratio between piperidine and the compound of formula (IV) is comprised from 0.58 to 0.97.

In an embodiment, optionally in combination with one or more features of the various embodiments described above or below, steps (a) and (b) of the process for the preparation of the pyrvinium methyl sulphate of formula (II) are performed in the presence of one or more solvents.

In an embodiment, optionally in combination with one or more features of the various embodiments described above or below, step (a) is performed in the presence of a solvent selected from the group consisting of C₁-C₄ hydrocarbon, C₃-C₆ cyclic ethers, C₁-C₃ alkyl-COO-C₁-C₄ alkyl, halo-C₁-C₄-hydrocarbons, and mixtures thereof. Examples of appropriate solvents for step (a) include toluene, tetrahydrofuran, dioxane, ethyl acetate, isopropyl acetate, dichloroethane and mixture thereof. In an embodiment, the solvent of step (a) is toluene.

In an embodiment, optionally in combination with one or more features of the various embodiments described above or below, step (a) is performed in the presence of a solvent in an amount comprised from 2 to 10 L of solvent in relation to the kilograms of the compound of formula (IV); preferably comprised from 3 to 5 L of solvent in relation to the kilograms of the compound of formula (IV); more preferably the amount of solvent is 3.5 L of solvent in relation to the kilograms of the compound of formula (IV). In an embodiment, optionally in combination with one or more features of the various embodiments described above or below, the step (a) is performed in the presence of toluene in an amount comprised from 2 to 10 L of toluene in relation to the kilograms of the compound of formula (IV); preferably comprised from 3 to 5 L of toluene in relation to the kilograms of the compound of formula (IV); more preferably the amount of solvent is 3.5 L of toluene in relation to the kilograms of the compound of formula (IV).

In an embodiment, optionally in combination with one or more features of the various embodiments described above or below, step (b) is performed in the presence of a solvent selected from the group consisting of C₁-C₄ alcohol, C₃-C₆ cyclic ethers; C₁-C₃ alkyl-COO-C₁-C₄ alkyl and mixtures thereof. Examples of appropriate solvents for step (b) include methanol, tetrahydrofuran, dioxane, ethyl acetate, isopropyl acetate. In a preferred embodiment, step (b) is performed in the presence of a solvent selected from the group consisting of methanol, tetrahydrofurane, ethyl acetate and mixtures thereof; preferably methanol.

In an embodiment, optionally in combination with one or more features of the various embodiments described above or below, step (b) is performed in the presence of a solvent in an amount comprised from 0.5 to 5 L of solvent in relation to the kilograms of the compound of formula (IV); preferably comprised from 1 to 3 L of solvent in relation to the kilograms of the compound of formula (IV); more preferably the amount of solvent is 1.4 L of solvent in relation to the kilograms of the compound of formula (IV). In an embodiment, optionally in combination with one or more features of the various embodiments described above or below, step (b) is performed in the presence of methanol in an amount comprised from 0.5 to 5 L of methanol in relation to the kilograms of the compound of formula (IV); preferably comprised from 1 to 3 L of methanol in relation to the kilograms of the compound of formula (IV); more preferably the amount of solvent is 1.4 L of methanol in relation to the kilograms of the compound of formula (IV).

In a particular embodiment, optionally in combination with one or more features of the various embodiments described above or below, step (a) is performed in the presence of toluene and step (b) is performed in the presence of methanol.

As it was mentioned above, the second aspect of the invention relates a process for the preparation of the crystalline Form A of pyrvinium pamoate which comprises: (c) slurring the crystalline Form III of pyrvinium pamoate with ethanol at reflux temperature for the necessary period of time to convert the crystalline Form III in crystalline Form A; and (d) cooling at a temperature comprised from 20°C to 30°C. In an embodiment, optionally in combination with one or more features of the various embodiments described above or below, step (c) of the process for the preparation of crystalline Form A of the pyrvinium pamoate is performed at a reflux temperature of ethanol for a period of time comprised from 1 to 5 h; preferably for 1 h. In an embodiment, optionally in combination with one or more features of the various embodiments described above or below, the process for the preparation of crystalline Form A of the pyrvinium pamoate further comprises (e) separating the crystalline Form A obtained in step (d); and (f) washing the crystalline Form A obtained in step (e) with ethanol. The crystalline Form A thus obtained is also part of the invention.

In an embodiment, optionally in combination with one or more features of the various embodiments described above or below, the process for the preparation of the crystalline Form A comprises preparing the crystalline Form III of pyrvinium pamoate as defined in the present invention; followed by the conversion of crystalline Form III in crystalline Form A as defined in the present invention.

As mentioned above, the third aspect of the invention relates to a crystalline Form A of pyrvinium pamoate characterized by having an X-ray diffractogram that comprises characteristic peaks at 2.8, 5.1, 7.2, 8.9, 9.6, 9.9, 15.9, 16.3, 17.9, and 21.0 ± 0.2 degrees 2 theta measured in an X-ray diffractometer with Cu Kα radiation (1.5418 Å). In an embodiment, the crystalline Form A of pyrvinium pamoate is characterized by having an X-ray diffractogram that further comprises characteristic peaks at 2.8, 5.1, 7.2, 8.9, 9.6, 9.9, 13.4, 14.3, 15.9, 16.3, 17.2, 17.9, 19.2, 19.8, 21.0,22.2,22.7, and 24.1 ± 0.2 degrees 2 theta measured in an X-ray diffractometer with Cu Kα radiation (1.5418 Å). More specifically, this crystalline Form A is characterized by exhibiting in the powder X-ray diffractogram a pattern of peaks, expressed in 2 theta units in degrees, 2θ (°), which is shown in following Table 1.

**Table 1: List of characteristic peaks obtained by X-ray diffractogram of the crystalline Form A of pyrvinium pamoate:**

| **Pos. [°2Th.]** | **Rel. Int. [%]** | **Pos. [°2Th.]** | **Rel. Int. [%]** |
|---|---|---|---|
| 2.8 | 21.9 | 16.3 | 9.2 |
| 5.1 | 7.5 | 17.2 | 6.8 |
| 7.3 | 5.6 | 17.9 | 100.0 |
| 8.9 | 32.1 | 19.2 | 12.0 |
| 9.6 | 35.1 | 19.8 | 10.1 |
| 9.9 | 18.3 | 21.0 | 16.8 |
| 13.4 | 5.2 | 22.2 | 9.5 |
| 14.3 | 6.8 | 22.7 | 12.6 |
| 15.9 | 22.7 | 24.1 | 13.1 |

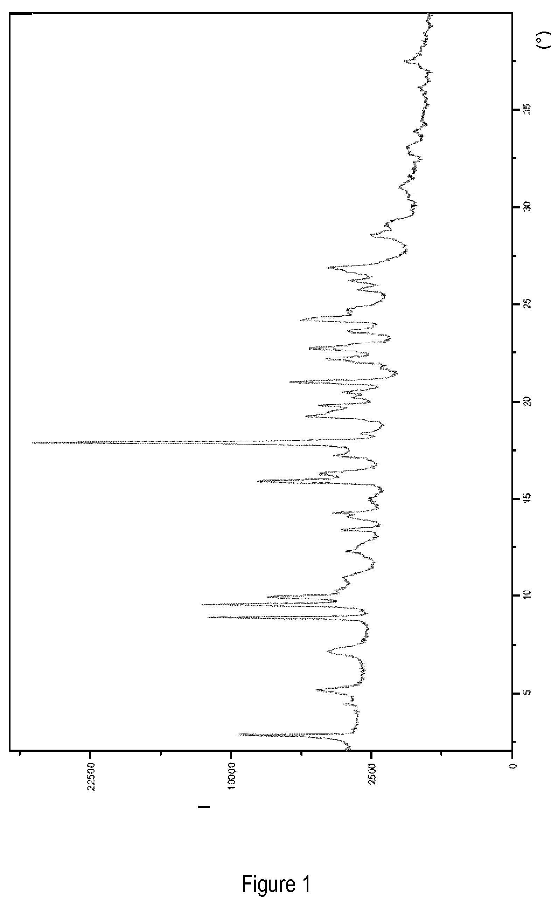

In an embodiment, the crystalline Form A of pyrvinium pamoate may be further characterized by having an X-ray diffractogram as shown in Fig. 1.

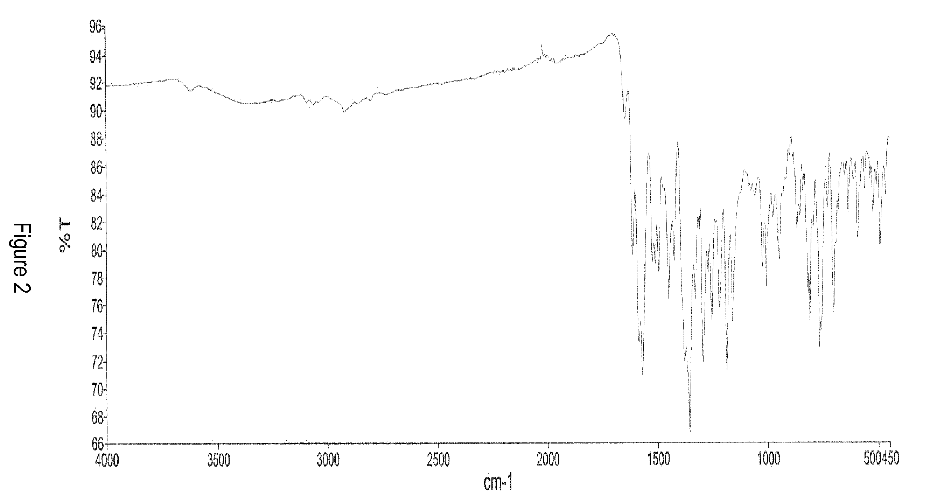

In a particular embodiment, the crystalline Form A of pyrvinium pamoate is characterized by having an infrared (IR) spectrum that shows the following peaks (±5 cm⁻¹): 1615, 1586, 1571, 1496, 1450, 1356, 1295, 1255, 1220, 1188, 1162, 1025, 1007, 948, 809, 766, and 703 cm⁻¹. In an embodiment, the crystalline Form A of pyrvinium pamoate is characterized by having the infrared (IR) spectrum as shown in Fig. 2.

In an embodiment, the chemical purity of the crystalline Form A of the pyrvinium pamoate of the present invention is equal to or higher than 99 area %; preferably equal to or higher than 99.5 area %; more preferably equal to or higher than area 99.8%; particularly equal to or higher than 99.95 area % measured by HPLC. In an embodiment, the polymorphic purity of the crystalline Form A of the pyrvinium pamoate is such that no other crystalline forms of the pyrvinium pamoate are detectable by X-ray powder diffraction measurement when using a X-ray diffractometer with Cu-Kα radiation λ=1.5406 Å.

An advantage of the crystalline Form A of the pyrvinium pamoate of the present invention lies in the fact that it is obtained with high yields and high purity; and it has the physical-mechanical properties that allow good manipulation for the preparation of solid pharmaceutical formulations meeting the strict pharmaceutical standards for their oral administration.

Another advantage of the crystalline Form A of the pyrvinium pamoate of the present invention is that allows eliminating and/or reducing the amount of impurities of the pyrvinium pamoate, particularly of crystalline Form III of the pyrvinium pamoate. Then, the crystalline Form A is useful as intermediate for the preparation of crystalline Form III of the pyrvinium pamoate.

The fourth aspect of the present invention relates to a process for the preparation of crystalline Form III of pyrvinium pamoate. In an embodiment, optionally in combination with one or more features of the various embodiments described above or below, the process for the preparation of crystalline Form III of pyrvinium pamoate comprises: (g) slurring the crystalline Form A of the pyrvinium pamoate with a mixture of water and ethanol at reflux temperature for the necessary period of time to convert the crystalline Form A in crystalline Form III; preferably for a period of time comprised from 15 to 20 h; (h) separating the crystalline Form III obtained in step (g) at a temperature comprised from 90°C to 95°C; and (i) washing the crystalline Form III obtained in step (h) first with hot water at a temperature comprised from 90°C to 95°C and then with ethanol.

In an embodiment, optionally in combination with one or more features of the various embodiments described above or below, the volume ratio between the water and the ethanol in step (g) is comprised from 15:1 to 10:1; preferably comprised from 14:1 to 11:1.

The fifth aspect of the present invention relates to a process for the purification of crystalline Form III of pyrvinium pamoate. The process comprises preparing crystalline Form A of pyrvinium pamoate from crystalline Form III by the process of the invention and then transforming crystalline Form A into crystalline Form III by the process as defined above. All the embodiments disclosed above for the preparation of the pyrvinium pamoate, crystalline form III and crystalline Form A also apply for the process for the purification of crystalline Form III of the fifth aspect of the invention.

The process for the purification of crystalline Form III of pyrvinium pamoate which comprises preparing crystalline Form A and then transforming crystalline Form A into crystalline Form III allows reducing the amount of total impurities from 0.66% to 0.24% by weight measured by HPLC. As it is shown in the Examples, this process particularly allows reducing the amount of the by-product (E)-2-[2-(2,5-dimethyl-1-phenyl-1 H-pyrrol-3-yl)-vinyl]-1-methyl-6-methylamino-quinolinium pamoate from 0.21% to 0.12% by weight measured by HPLC. Thus, the chemical purity of the crystalline Form III of pyrvinium pamoate obtained by the process of the fifth aspect of the invention is equal to or higher than 99 area %; preferably equal to or higher than 99.5 area %; more preferably equal to or higher than 99.8 area %; particularly equal to or higher than 99.95 area % measured by HPLC. In an embodiment, the polymorphic purity of the crystalline Form III of the pyrvinium pamoate is such that no other crystalline forms of the pyrvinium pamoate are detectable by X-ray powder diffraction measurement when using a X-ray diffractometer with Cu-Kα radiation λ=1.5406 Å.

As mentioned above, the sixth aspect of the invention relates to the crystalline Form III of pyrvinium pamoate characterized by having an X-ray diffractogram that comprises characteristic peaks at 6.0, 8.6, 9.5, 10.3, 10.5, 14.3, 14.9, 16.6, 17.4, 18.1, 19.1, 19.7, 20.6, 21.5, 22.0, 22.6, 23.6, 24.9 ± 0.2 degrees 2 theta measured in an X-ray diffractometer with Cu Kα radiation (1.5418 Å). In an embodiment, the crystalline Form III of pyrvinium pamoate is characterized by having an X-ray diffractogram that further comprises characteristic peaks at 6.0, 8.6, 9.5, 9.9, 10.3, 10.5, 10.7, 11.2, 12.9, 14.3, 14.5, 14.9, 16.6, 16.9, 17.4, 18.1, 19.1, 19.7, 20.0, 20.6, 21.5, 22.0, 22.6, 23.6, 24.5, 24.9, and 26.4 ± 0.2 degrees 2 theta measured in an X-ray diffractometer with Cu Kα radiation (1.5418 Å). More specifically, this crystalline Form III is characterized by exhibiting in the powder X-ray diffractogram a pattern of peaks, expressed in 2 theta units in degrees, 2θ (°), which is shown in the following Table 2.

**Table 2: List of characteristic peaks obtained by X-ray diffractogram of the crystalline Form III of pyrvinium pamoate:**

| **Pos. [°2Th.]** | **Rel. Int. [%]** | **Pos. [°2Th.]** | **Rel. Int. [%]** |
|---|---|---|---|
| 6.0 | 36.7 | 17.4 | 45.7 |
| 8.6 | 16.5 | 18.1 | 43.3 |
| 9.5 | 100.0 | 19.1 | 16.6 |
| 9.9 | 28.3 | 19.7 | 25.3 |
| 10.3 | 39.0 | 20.0 | 25.5 |
| 10.5 | 48.0 | 20.6 | 16.4 |
| 10.7 | 24.1 | 21.5 | 14.0 |
| 11.2 | 11.0 | 22.0 | 19.3 |
| 12.9 | 4.9 | 22.6 | 33.9 |
| 14.3 | 77.6 | 23.6 | 17.2 |
| 14.5 | 13.3 | 24.5 | 16.9 |
| 14.9 | 22.9 | 24.9 | 27.7 |
| 16.6 | 36.4 | 26.4 | 17.9 |
| 16.9 | 22.7 | | |

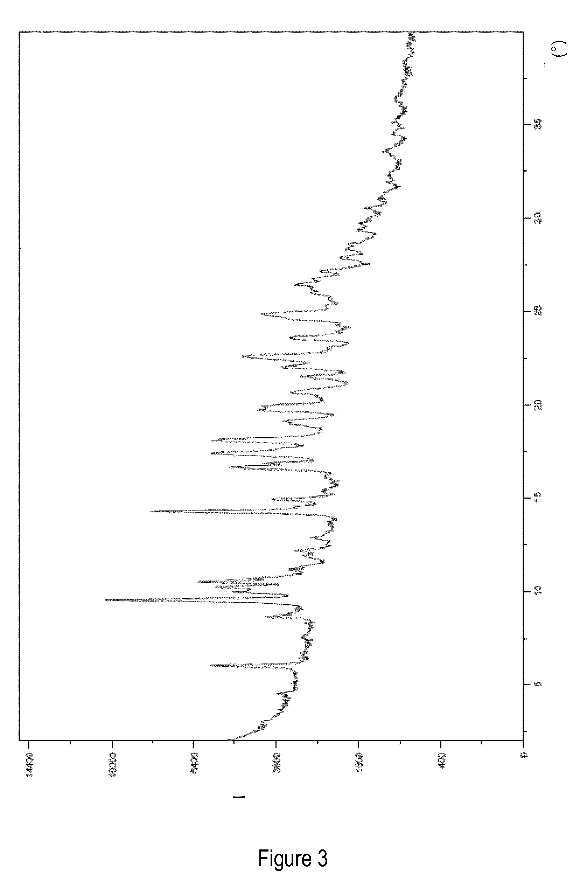

In an embodiment, the crystalline Form III of pyrvinium pamoate may be further characterized by having an X-ray diffractogram as shown in Fig. 3.

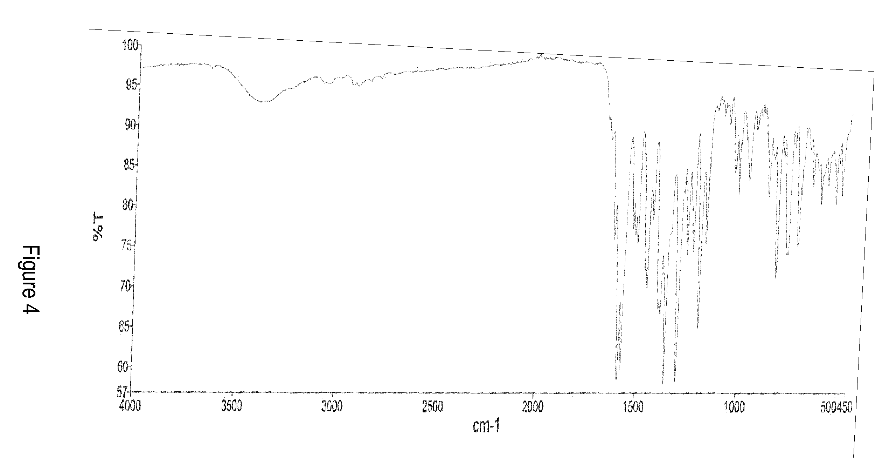

In a particular embodiment, the crystalline Form III of pyrvinium pamoate is characterized by having an infrared (IR) spectrum that shows the following peaks (±5 cm⁻¹): 3360 (broad peak), 1618, 1590, 1573, 1500, 1450, 1382, 1357, 1298, 1254, 1224, 1192, 1162, 1031, 1008, 957, 859, 813, 755, and 707 cm⁻¹. In an embodiment, the crystalline Form III of pyrvinium pamoate is characterized by having the infrared (IR) spectrum as shown in Fig. 4.

As it was mentioned above, the seventh aspect of the present invention relates to a pharmaceutical composition comprising a therapeutic effective amount either of the crystalline Form A or of the crystalline Form III of pyrvinium pamoate as defined herein, together with one or more pharmaceutically acceptable excipients or carriers. In an embodiment, optionally in combination with one or more features of the various embodiments described above or below, the pharmaceutical composition can be in form of an oral pharmaceutical composition. The compositions of the present invention can be prepared according to methods well known in the state of the art. The appropriate excipients and/or carriers, and their amounts, can readily be determined by those skilled in the art according to the type of formulation being prepared.

It also forms part of the invention the crystalline Forms A and III of pyrvinium pamoate as defined herein for use as anthelmintic, particularly for use in the treatment of enterobiasis. It forms also part of the invention the use of the crystalline Forms A and III of pyrvinium pamoate for the manufacture of a medicament for the treatment of enterobiasis. This aspect could be also formulated as a method for the treatment of enterobiasis, comprising administering a pharmaceutically effective amount of the crystalline Forms A and III of pyrvinium pamoate, together with pharmaceutically acceptable excipients or carriers, to a subject in need thereof, including a human.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### General considerations

The chemical displacements of the proton (¹H) and of the carbon (¹³C) were obtained with a Bruker nuclear magnetic resonance spectrometer. The chemical displacements are given in ppm, referenced internally to the deuterated solvent.

The molecular mass has been confirmed through the direct infusion into Waters micromass ZQ 2000 spectrometer with an electrospray probe (ES).

The water content was carried out on an 831 Karl Fischer Coulometer with a 774 Oven Sample Processor. Solid sample is heated in the oven; the released water is transported in a dry stream of carrier gas to the Karl Fischer titration cell with a generator electrode without diaphragm, where it is determined with Hydranal Coulomat E reactive.

Powder X-Ray diffraction (PXRD) analyses were performed by sandwiching the powder samples between polyester films of 10 micrometres of thickness or polyamide (kapton) films of 15 micrometres of thickness and analysed in a PANalytical X'Pert PRO MPD q/q powder diffractometer of 240 millimetres of radius, in a configuration of convergent beam with a focalizing mirror and a flat sample transmission geometry, in the following experimental conditions: Cu Kα radiation (A = 1.5418 Å); Work power: 45 kV and 40 mA; Incident beam slits defining a beam height of 0.4 millimetres; Incident and diffracted beam 0.02 radians Soller slits; PlXcel detector: Active length = 3.347 °; 2θ/θ scans from 2 to 40 °2θ with a step size of 0.026 °2θ and a measuring time of 76 seconds per step. The X-ray diffractogram shows the powder X-ray diffraction pattern (intensity (counts) vs. 2-theta angle (°)) of the crystalline Form of pyrvinium pamoate salt of formula (I).

Fourier Transform Infrared - Attenuated Total Reflectance (FT-IR-ATR) spectra were registered on a Perkin Elmer Spectrum Two FT-IR spectrometer with universal attenuated total reflectance (UATR) sampling accessory (UATR Two). Sample is placed on the ATR plate and the measure is carried out in the 4000-450 cm-1 range. The term "IR or IR spectrum/ spectra" when used in the context refers to spectra registered in the conditions mentioned above.

High-pressure liquid chromatography (HPLC)._HPLC analyses were performed with a Waters 2695 and Waters 1525 Separation Module with a UV detector UV PDA 2996 or Dual 2487; using the following conditions: Solvents: ammonium formate (analysis grade); formic acid (analysis grade); acetonitrile (HPLC grade); methanol (HPLC grade); and purified water (milli-Q grade); Column: XBridge C18 (waters; 150x4.6 mm) 3,5 µm; Flow: 0,7 mL/min; Injection volume: 10 µL;·Injection temperature: 25 °C;·UV detection: 344 nm; Sample dissolved in Acetonitrile : 10mM ammonium formate (pH 3.3);·Gradient:

| time (min) | % acetonitrilo | %ammonium formate | curve |
|---|---|---|---|
| 0 | 35 | 65 | |
| 27 | 60 | 40 | 6 |
| 30 | 75 | 25 | 1 |
| 35 | 35 | 65 | 1 |

### Example 1. Preparation of crystalline Form III of pyrvinium pamoate

### Step 1. Preparation of pyrvinium methyl sulphate of formula (II)

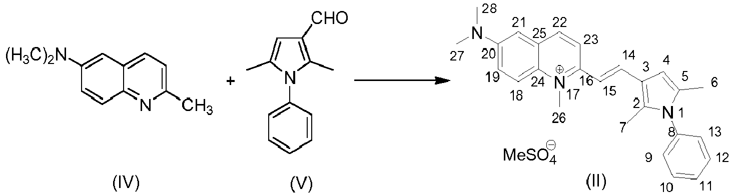

A mixture of 6-dimethylaminoquinaldine of formula (IV) (150 kg) in toluene (525 L) are heated to 45-50°C for 30 minutes until complete solution. After cooling to 25-30°C, dimethyl sulphate (105 kg) is slowly added and the mixture is maintained to 75-85°C for 1 hour. Then, a hot solution of 2,5-dimethyl-1-phenyl-1 H-pyrrole-3-carbaldehyde of formula (V) (126.45 kg) in methanol (210 L). Then, piperidine (48 kg) is added, and the resulting mixture is refluxed for about 2.5 h and the solvent distilled under vacuum to obtain pyrvinium methyl sulphate of formula (II) as a pasty solid. Once the obtained pasty is cooled, methanol (114 L) and acetone (380.7 L) is added and the mixture is refluxed for about 1 hour. Then, the content of the reactor is cooled to 0-10°C at least 2 hours, centrifuged and the solid obtained is washed with acetone (40 L) to obtain the pyrvinium methyl sulphate of formula (II) (240 kg; yield 60,8% and 77,2% with respect to compounds (IV) and (V), respectively).

### Pyrvinium methyl sulphate of formula (II):

**IR** (ATR, cm⁻¹): 1616, 1585 y 1571 (arom.), 1374, 1354, 1228 y 1057(CH₃O-SO₃-), 1012, 707. **MS:** 382 (M+)
**¹H NMR (300 MHz; DMSO):** 8.50 (d, J=9 Hz, 1 H, CH H(22)), 8.42 (d, J=9 Hz, 1 H, CH H(23)), 8.21 (d, J= 9 Hz, 1H, CH H(18)), 8.06 (d, J=15 Hz, 1H, CH H(14) o HC(15)), 7.57 (m, 4H, CH H(11)+H(19)+H(10)+H(12)), 7.35 (d, J=6 Hz, 2H, CH H(9)+H(13)), 7.20 (j, J=2,7 Hz, 1H, CH H(21)), 7.19 (d, J=15 Hz, 1H, CH HC(15) o H(14)), 6.72 (s, 1H, CH HC(4)), 4.35 (s, 3H, CH₃ MeSO₄⁻ o H(26)), 3,37 (s, 3H, CH₃ H(26) o MeSO₄⁻), 3.09 and 3.02 (s, 6H CH₃ H(27)+H(28)), 2.23 (s, 3H, CH₃ H(6) o H(7)), 2.00 (s, 3H, CH₃ H(7) o H(6)),

### Step 2. Preparation of pyrvinium pamoate

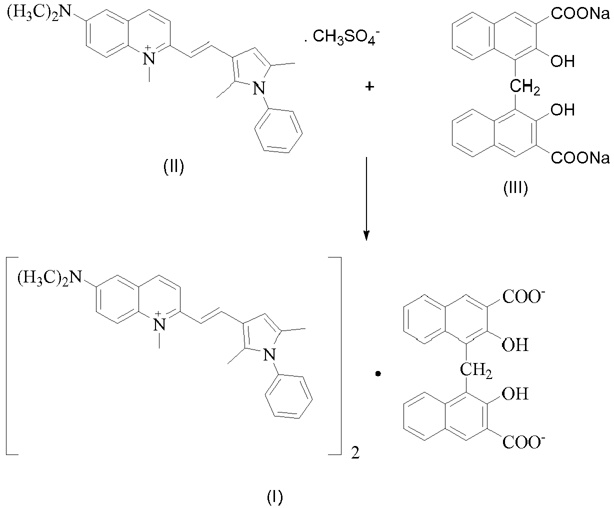

A mixture of pyrvinium methyl sulphate of formula (II) obtained in step 1 (40 kg) in water (1000 L) is refluxed for about 1 hour. Then, a hot solution of disodium pamoate of formula (III) (19.3 kg) in water (130 L) and ammonia (7 L) is slowly added over the contents of the reactor. After heating to reflux for 9-10 hours, the mixture is cooled to 50-60°C, ethanol (75 L) is added, again is refluxed for about 1 hour and centrifuged in hot. Then, the solid obtained is first washed with hot water (1000 L) and then with ethanol (35 L) given pyrvinium pamoate, which is dried under vacuum for 14-17 hours at 84-88°C and finally milled to obtained the crystalline Form III of pyrvinium pamoate having a water content measured by the Karl Fisher method equal to or less than 6.0% by weight (46 kg; yield 100%, KF 4.5%). The crystalline Form III of pyrvinium pamoate has a purity by HPLC of 99.34 area %, a content of the impurity of formula (E)-2-[2-(2,5-dimethyl-1-phenyl-1 H-pyrrol-3-yl)-vinyl]-1-methyl-6-methylamino-quinolinium pamoate of 0.21 area % purity by HPLC; and a content of unknown impurities of 0.45 area % purity by HPLC.

### Pyrvinium pamoate:

**MS:** 382.3 (M+)
**¹H-NMR (300 MHz, DMSO) δ (ppm):** 8.49 (d, J= 9 Hz, 2H, H₂₂), 8.43 (d, J= 9 Hz, 2H, H₂₃), 8.12 (2d, J= 10 Hz, 4H, H₁₈,H₃₇), 8.10 (s, 2H, H₃₃), 8.08 (d, J=15 Hz, 2H, H₁₅), 7.6-7.4 (m, 10H, H₁₀, H₁₁, H₁₂, H₁₉, H₃₄), 7.37-7.34 (2d, 4H, H₉, H₁₃), 7.21 (d, J=15 Hz, 2H H₁₄), 7.20 (s, 2H, H₂₁), 7.06 (m, 2H, H₃₆), 6.96 (m, 2H, H₃₅), 6.72 (s, 2H, H₄), 4.64 (s, 2H, H₂₉(CH₂)), 4.34 (s, 6H, H₂₆ (CH₃)), 3.08 (s, 12H, H₂₇ and H₂₈ (CH₃)), 2.23 (s, 6H, H₇ (CH₃), 2.00 (s, 6H, H₆ (CH₃)).

### (E)-2-[2-(2,5-dimethyl-1-phenyl-1H-pyrrol-3-yl)-vinyl]-1-methyl-6-methylamino-quinolinium pamoate:

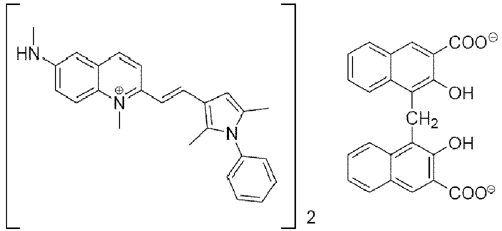

**¹H-NMR (300 MHz, CDCl₃) δ (ppm):** 8.48 (d, J= 9, 1 H, HC(22)), 8.41 (d, J= 9, 1 H, HC(23)), 8.20-8.12 (m+s, 3H, HC(18)+HC(34)+HC(37)), 8.04 (d, J= 15, 1 H, HC(15)), 7.58 (m, 5H, HC(10), HC(11), HC(12), HC(19), HC(34)), 7.38 (m, 2H, HC(9), HC(13)), 7.20 (d, J=15, 1H HC(14), 7.07 (t, J=7 Hz, 1H, HC(35)), 6.95 (m, 2H, HC(19)+HC(21)), 6.82 (m, 1H, HC(Ar)), 6.70 (s, 1H, HC(4), 4.64 (s, 2H, H₂C(29), 4.33 (s, 3H, H₂C(26), 3.34 (s, H₂O), 2,82 (d, J=5 Hz, 3H, H₃C(27)), 2.22 (s, 3H, H₃C(7)), 2.00 (s, 3H, H₃C(6)).

### Example 2. Purification of the crystalline Form III of pyrvinium pamoate

### Step 1. Preparation of crystalline Form A of pyrvinium pamoate

Crystalline Form III of pyrvinium pamoate obtained in step 2 of Example 1 (60g) and ethanol (1500 mL) were mixed in a reactor. The resultant mixture is refluxed for at least 1 hour. Then, the content of the reactor is cooled to 20-30°C and it is maintained at that temperature for at least 1 hour, and after that time, the ethanol was centrifuged. Then, the solid obtained was washed with ethanol (300 mL). Crystalline Form A of pyrvinium pamoate was obtained as a maroon solid. The crystalline Form A of pyrvinium pamoate has a purity by HPLC of 99.69 area %, a content of the impurity of formula (E)-2-[2-(2,5-dimethyl-1-phenyl-1 H-pyrrol-3-yl)-vinyl]-1-methyl-6-methylamino-quinolinium pamoate of 0.12 area % purity by HPLC, and a content of unknown impurities of 0.19% purity by HPLC.

### Step 2. Conversion of crystalline Form A to crystalline Form III of pyrvinium pamoate

Crystalline Form A of pyrvinium pamoate obtained in step 1 of Example 2, ethanol 96° (120 mL) and water (1500 mL) were mixed in a reactor. The resultant mixture is refluxed for 15 hour. Then, at the refluxed temperature, the content of the reactor was centrifuged. Then, the solid obtained was washed with hot water (1500mL) and with ethanol (120 mL). Crystalline Form III of pyrvinium pamoate was obtained as red solid and dried in an oven under vacuum at a 85°C for 15 h (53.5g, two steps overall yield 89%, KF 5.8%). The crystalline Form III of pyrvinium pamoate has a purity by HPLC of 99.76 area %, a content of the impurity of formula (E)-2-[2-(2,5-dimethyl-1-phenyl-1H-pyrrol-3-yl)-vinyl]-1-methyl-6-methylamino-quinolinium pamoate of 0.12 area % purity by HPLC, and a content of unknown impurities of 0.12 area % purity by HPLC.

## Claims

1. A process for the preparation of pyrvinium pamoate salt of formula (I), 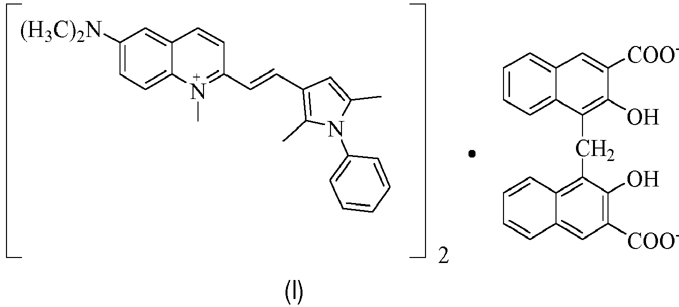 which comprises reacting pyrvinium methyl sulphate salt of formula (II), 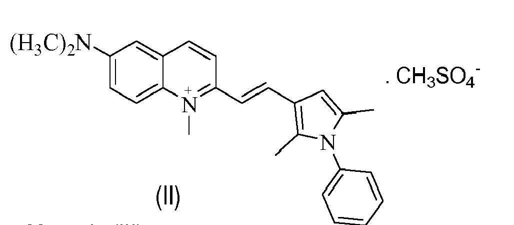 with disodium pamoate of formula (III), 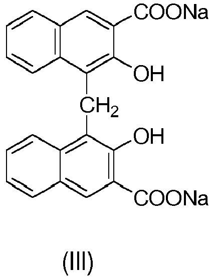 optionally, in the presence of a base.

2. The process according to claim 1, wherein the base is ammonia.

3. The process according to any of the claims 1-2, wherein the molar ratio between the compound of formula (II) and the compound of formula (III) is comprised from 1.33 to 2.00.

4. The process according to any of the claims 1-3, wherein the process is performed in the presence of a mixture of water and C₁-C₄ alcohol and then the process renders the crystalline Form III of pyrvinium pamoate salt of formula (I).

5. The process according to any of the claims 1-4, wherein the process further comprises first preparing the compound of formula (II) by:
a) reacting a compound of formula (IV), 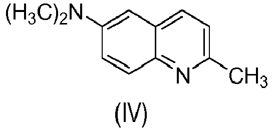 with dimethyl sulphate; and
b) reacting the mixture obtained in step a) with a compound of formula (V) 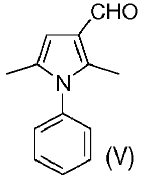 in the presence of a base.

6. The process according to claim 5, wherein:
step a) is performed in the presence of a solvent selected from the group consisting of C₁-C₄ hydrocarbons, C₃-C₆ cyclic ethers, C₁-C₃ alkyl-COO-C₁-C₄ alkyl, halo-C₁-C₄-hydrocarbons; and mixtures thereof; and
step b) is performed in the presence of a solvent selected from the group consisting of C₁-C₄ alcohol, C₃-C₆ cyclic ethers; C₁-C₃ alkyl-COO-C₁-C₄ alkyl; and mixtures thereof.

7. The process according to any of the claims 5-6, wherein:
step a) is performed in the presence of toluene; and
step b) is performed in the presence of methanol.

8. The process according to any of the claims 5-7, wherein the base of step b) is piperidine.

9. A process for the preparation of a crystalline Form A of pyrvinium pamoate salt of formula (I) which comprises:
(c) slurring the crystalline Form III of the pyrvinium pamoate salt of formula (I) with ethanol at reflux temperature for the necessary period of time to convert the crystalline Form III in crystalline Form A; and
(d) cooling at a temperature comprised from 20°C to 30°C.

10. The process for the preparation of the crystalline Form A of pyrvinium pamoate salt of formula (I) according to claim 9, wherein step (c) is performed for a period of time comprised from 1 to 5 h.

11. A process for the preparation of crystalline Form III of pyrvinium pamoate salt of formula (I) which comprises:
(g) slurring the crystalline Form A of pyrvinium pamoate salt of formula (I) with a mixture of water and ethanol at reflux temperature for the necessary period of time to convert the crystalline Form A in crystalline Form III;
(h) separating the crystalline Form III obtained in step (g) at a temperature comprised from 90°C to 95°C; and
(i) washing the crystalline Form III obtained in step (h) first with hot water at a temperature comprised from 90°C to 95°C and then with ethanol.

12. A process for the purification of crystalline Form III of pyrvinium pamoate salt of formula (I) which comprises preparing crystalline Form A of pyrvinium pamoate salt of formula (I) from crystalline Form III obtained by the process of any of the claims 9-10 and then transforming crystalline Form A into crystalline Form III by the process of claim 11.

13. A crystalline Form A of pyrvinium pamoate salt of formula (I) **characterized by** having an X-ray diffractogram that comprises characteristic peaks at 2.8, 5.1, 7.2, 8.9, 9.6, 9.9, 15.9, 16.3, 17.9, 21.0 ± 0.2 degrees 2 theta measured in an X-ray diffractometer with Cu Kα radiation (1.5418 Å).

14. A crystalline Form III of pyrvinium pamoate salt of formula (I) **characterized by** having an X-ray diffractogram that comprises characteristic peaks at 6.0, 8.6, 9.5, 10.3, 10.5, 14.3, 14.9, 16.6, 17.4, 18.1, 19.1, 19.7, 20.6, 21.5, 22.0, 22.6, 23.6, and 24.9 ± 0.2 degrees 2 theta measured in an X-ray diffractometer with Cu Kα radiation (1.5418 Å).

15. A pharmaceutical composition comprising any of the crystalline Forms of pyrvinium pamoate salt of formula (I) defined in any of the claims 13-14, together with one or more pharmaceutically acceptable excipients or carriers.
